# EUROPEAN PATENT APPLICATION

(11) **EP 3 984 627 A1**
(43) Date of publication of application: **20.04.2022**
(21) Application number: 20306241.9
(22) Date of filing: 19.10.2020
(51) Int. Cl.: B01F 13/00, B01F 5/04, B01F 5/06, B01D 9/00

(54) **APPARATUS AND METHOD FOR THE PURIFICATION OF BIOMOLECULES**

(71) Applicant: NOVASEP EQUIPMENT SOLUTIONS, 54340 Pompey (FR)
(72) Inventor: VALÉRY, Eric, 54420 Saulxures-lès-Nancy (FR); PONS-ROYO, Maria del Carme, 54000 Nancy (FR)
(74) Representative: Bandpay & Greuter

(57) **Abstract**

The invention relates to an apparatus for the purification of biomolecules, comprising an injection device (1) comprising an injection channel (7), the injection channel being provided with at least one inlet (3) for a precipitation agent composition and a plurality of outlets (Si) on the external surface of the injection channel; and a mixing device (2) comprising a mixing channel (4) provided with at least one main inlet (5) for a composition to be purified and at least one main outlet (6), as well as a plurality of secondary inlets; wherein the injection device (1) and the mixing device (2) are coupled such that each of the plurality of outlets (Si) of the injection device (1) is connected to a respective secondary inlet of the mixing channel (4).

The invention further relates to a set comprising said apparatus, a composition comprising at least one biomolecule and at least one impurity, and a composition comprising at least one precipitation agent, and to a method for the purification of biomolecules using said apparatus.

## Description

The project leading to this application has received funding from the European Union's Horizon 2020 research and innovation program under the Marie Sktodowska-Curie grant agreement No 812909.

### TECHNICAL FIELD

The present invention relates to an apparatus for the purification of biomolecules. The present invention also relates to a method for the purification of biomolecules carried out in said apparatus.

### TECHNICAL BACKGROUND

Purification of biomolecules such as proteins or antibodies is usually carried out by chromatography techniques. However, over the last years, purification by precipitation is a technique that has continuously gained ground due to advantages such as the cost of the procedure. The technique of precipitation consists in mixing a precipitation agent with a mixture comprising the biomolecule of interest in order to precipitate either the biomolecule or one or more impurities. In this way, the biomolecule of interest can be isolated. More particularly, the precipitation agent promotes the appearance of a solid phase which can later be separated from the remaining liquid. This phenomenon begins with the formation of solid nuclei, which, as the phenomenon continues, will increase in size during a growth phase.

Generally, such process is carried out in a reactor comprising two conduits joined by a fluid connection before passing through a static mixer and then into a tube the volume of which makes it possible to maintain a desired residence time in the reactor. However, when there is a change of scale, mixing problems may appear: for example as the diameter of the conduit bringing the precipitation agent increases, there can be important local oversaturation at the mixing point of the precipitation agent and the flow of biomolecule to be treated. Such oversaturation may not only promote the nucleation phenomenon over the growth phenomenon (which makes filtration more difficult) but also cause the sudden appearance of a large mass of solid, of which agglomerates can trap the liquid phase, and therefore generate either a loss of purity or a loss of yield.

In order to overcome oversaturation issues, it has been proposed to use multiple pumps in order to inject the precipitation agent. However, this increases the complexity of the apparatus used for the purification.

The article *"*Continuous Plug Flow Crystallization of Pharmaceutical Compounds" of Alvarez A. J. et al. (Crystal Growth & Design, 2010, vol.10, 2219-2228) relates to a strategy of multiple points of addition of antisolvent along a crystallizer comprising ketoconazole, flufenamic acid or L-glutamic acid, in order to control the size of the crystals.

The article *"*Continuous precipitation of process related impurities from clarified cell culture supernatant using a novel coiled flow inversion reactor (CFIR)" of Kateja N. et al. (Biotechnology Journal, 2016, vol.10, 1320-1331) relates to the use of a coiled flow inversion reactor into a continuous bioprocess train for performing continuous precipitation of clarified cell culture supernatant.

Document WO 2016/116947 relates to a coiled flow inversion reactor for carrying out continuous protein refolding reactions in-vitro by dilution method.

The article *"*Multiple Feeding Strategy for Phase Transformation of GMP in Continuous Couette-Taylor Crystallizer" of Nguyen et al. (Crystal Growth & Design, 2012, vol.12, 2780-2788) relates to a continuous Couette-Taylor crystallizer with a multiple feed mode which makes it possible to promote phase transformation of guanosine 5-monophosphate.

The article *"*Oscillatory Flow Reactors (OFRs) for Continuous Manufacturing and Crystallization" of McGlone T. et al. (Organic Process Research & Development, 2015, vol.19, 1186-1202) provides a brief overview of OFR technology before outlining the operating principles and summarizing applications, emphasizing the use for controlled continuous crystallization.

The article *"*Continuously Seeded, Continuously Operated Tubular Crystallizer for the Production of Active Pharmaceutical Ingredients" of Eder R. J. P. et al. (Crystal Growth & Design, 2010, vol.10, 2247-2257) relates to a continuously operated tubular crystallizer system with which allows the crystallization of active pharmaceutical ingredients (APIs) under controlled conditions.

The article *"*Process Intensification through Continuous Spherical Crystallization Using an Oscillatory Flow Baffled Crystallizer (OFBC)" of Peña R. et al. (Crystal Growth & Design, 2017, 17, 9, 4776-4784) relates to a study based on performing crystallization/spherical agglomeration in an oscillatory flow baffled crystallizer (OFBC).

There is thus a need for an apparatus which is adaptable in multiple scales and which makes it possible to efficiently purify a biomolecule of interest while avoiding issues related to oversaturation and while maintaining a simple configuration.

### SUMMARY OF THE INVENTION

It is a first object of the invention to provide an apparatus for the purification of biomolecules, comprising:
- an injection device comprising an injection channel, the injection channel being provided with at least one inlet for a precipitation agent composition and a plurality of outlets on the external surface of the injection channel; and
- a mixing device comprising a mixing channel provided with at least one main inlet for a composition to be purified and at least one main outlet, as well as a plurality of secondary inlets;
wherein the injection device and the mixing device are coupled such that each of the plurality of outlets of the injection device is connected to a respective secondary inlet of the mixing channel.

According to some embodiments, the injection channel comprises from 2 to 40 outlets, and preferably from 2 to 20 outlets.

According to some embodiments, the outlets are distributed substantially linearly on the external surface of the injection channel and are preferably evenly spaced.

According to some embodiments, the outlets are grouped as distinct injection zones distributed along the injection channel.

According to some embodiments, the mixing device is configured as a coiled conduit.

According to some embodiments, the mixing channel is formed of a plurality of intersecting linear segments.

According to some embodiments, the apparatus comprises only one pump connected to the injection device.

It is a second object of the invention to provide a set comprising the apparatus described above, a composition comprising at least one biomolecule and at least one impurity, and a composition comprising at least one precipitation agent.

According to some embodiments, the biomolecule is chosen from a protein and a nucleic acid, and is preferably an antibody; and/or wherein the impurity is chosen from double stranded DNA, host cell proteins, high molecular weight impurities, low molecular weight impurities, and antibody fragments or aggregates; and/or wherein the precipitation agent is chosen from caprylic acid, polyethylene glycol, calcium chloride, zinc chloride, ethanol, isopropanol and combinations thereof.

It is a third object of the invention to provide a method for the purification of biomolecules using the apparatus described above, comprising:
- the injection of a composition comprising at least one biomolecule and at least one impurity into the mixing channel via the main inlet;
- the injection of a composition comprising at least one precipitation agent from the injection device into the mixing channel via the plurality of outlets of the injection device; and
- the precipitation of one of the at least one biomolecule or the at least one impurity in the mixing device.

According to some embodiments, both the injection of the composition comprising the biomolecule and the impurity and the injection of the composition comprising the precipitation agent are continuous.

According to some embodiments, the biomolecule is chosen from protein and a nucleic acid and is preferably an antibody; and/or wherein the impurity is chosen from double stranded DNA, host cell proteins, high molecular weight impurities, low molecular weight impurities and antibody fragments or aggregates.

According to some embodiments, the precipitation agent is chosen from caprylic acid, polyethylene glycol, calcium chloride, zinc chloride, ethanol, isopropanol, and combinations thereof.

According to some embodiments, the method further comprises a step of injecting into the mixing channel a solubilization agent or a buffer solution via at least one additional inlet of the mixing channel.

According to some embodiments, the method further comprises a step of injecting a composition comprising at least one additional precipitation agent into the mixing channel via at least two outlets of an additional injection device.

The present invention makes it possible to address the need expressed above. In particular, the invention provides an apparatus which is adaptable in multiple scales and which makes it possible to efficiently purify a biomolecule of interest while avoiding issues related to oversaturation and while maintaining a simple configuration.

This is achieved by the apparatus of the present invention, and more particularly by the combination of an injection device comprising an injection channel, the injection channel being provided with at least one inlet and a plurality of outlets, and of a mixing device comprising a mixing channel comprising at least one main inlet and at least one main outlet. The outlets of the injection device are located on the external surface of the injection channel and are connected to respective secondary inlets of the mixing channel of the mixing device. This makes it possible to distribute a precipitation agent injected through the inlet of the injection device and via the outlets of the injection device into the channel of the mixing device, at different locations of the mixing device. Thus, it allows a continuous and controlled distribution of the precipitation agent in the mixing device (which controls and optimizes the precipitation of the biomolecule of interest or of the one or more impurities) and limits issues related to oversaturation of the precipitation agent. Furthermore, it is possible to use a single pump (instead of a plurality of pumps, each pump being connected to a distinct outlet of the injection device) to control the injection of the precipitation agent through the different outlets and into the mixing channel of the mixing device.

In addition, the present invention provides different configurations of the apparatus, which makes it possible to implement the purification method in a variety of scales.

### BRIEF DESCRIPTION OF THE DRAWINGS

**Figure 1** schematically represents an injection device according to one embodiment of the present invention.
**Figure 2** schematically represents an injection device according to one embodiment of the present invention.
**Figure 3** schematically represents an injection device according to one embodiment of the present invention.
**Figure 4** schematically represents an injection device according to one embodiment of the present invention.
**Figure 5** schematically represents a mixing device according to one embodiment of the present invention.
**Figure 6** schematically represents the mixing channel of a mixing device according to one embodiment of the present invention.
**Figure 7** schematically represents the mixing channel of a mixing device according to one embodiment of the present invention.
**Figure 8** schematically represents the mixing channel of a mixing device according to one embodiment of the present invention.
**Figure 9** schematically represents the mixing channel of a mixing device according to one embodiment of the present invention.
**Figure 10** schematically represents the mixing channel of a mixing device according to one embodiment of the present invention.
**Figure 11** schematically represents the mixing channel of a mixing device according to one embodiment of the present invention.
**Figures 12A** to **12G** schematically represent the apparatus according to one embodiment of the present invention.
**Figure 13** shows an example of a precipitation curve. The concentration of a biomolecule in the liquid phase is plotted as a function of the amount of precipitation agent added. The concentration of the biomolecule (g/L) can be read on the Y-axis and the amount of precipitation agent added (%) can be read on the X-axis.

### DESCRIPTION OF EMBODIMENTS

The invention will now be described in more detail without limitation in the following description.

### Apparatus

The apparatus of the present invention allows the purification of at least one biomolecule of interest from a composition comprising at least one biomolecule of interest and at least one impurity. More particularly, the apparatus of the present invention allows the precipitation of the least one biomolecule of interest or of the at least one impurity. In the context of the present invention, the term *"precipitation"* means the formation of a solid phase in the liquid phase. Crystallization in particular is to be considered as a form of precipitation for the purpose of the present invention.

The apparatus of the present invention comprises an injection device 1 which carries out the injection of at least one precipitation agent and a mixing device 2 which carries out the mixing of the composition comprising at least one biomolecule of interest and at least one impurity with the at least one precipitation agent, the devices being connected together.

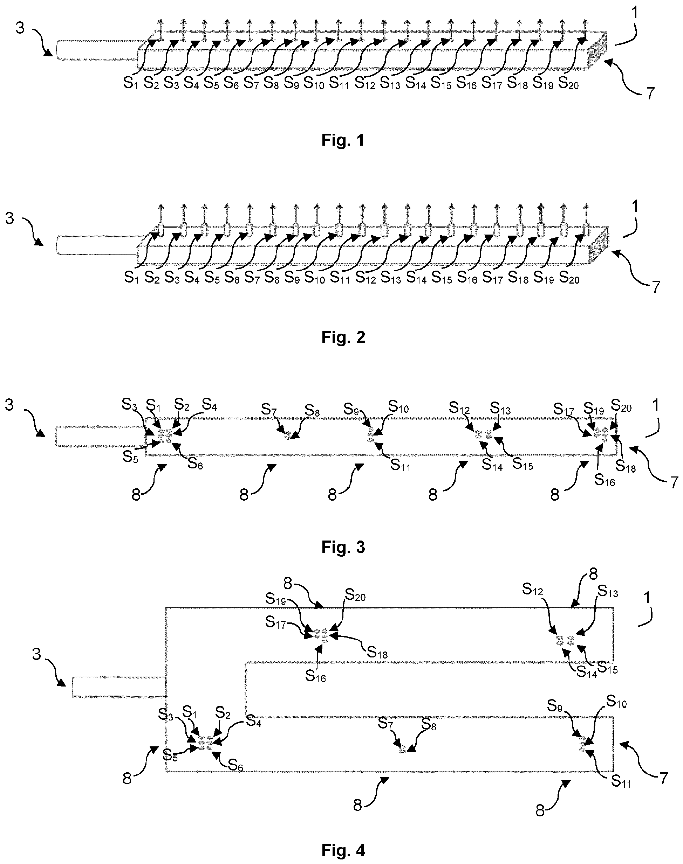

The injection device 1 can be described by making reference to **figures 1** to **4****.** The injection device 1 comprises an injection channel 7 which is provided with at least one inlet 3 and a plurality of outlets Si on the external surface of the injection channel 7. By *"channef'* is meant a tubular or cylindrical passage formed in the injection device 1 for the flow of fluid from the inlet 3 to the outlets Si.

According to some embodiments, the injection channel 7 is substantially linear (such as illustrated for example in **figures 1** to **3**)**.**

According to other embodiments, the injection channel 7 is branched, as illustrated in **figure 4****.** In this case, the fluid from the inlet 3 is distributed into two or more channel branches, each branch comprising one or more outlets Si.

The injection channel 7 can have for instance a rectangular or square or circular cross-section (perpendicular to the main direction of flow). A rectangular cross-section is illustrated in **figure 1****.**

The injection channel 7 may have an internal hydraulic diameter from 0.2 mm to 10 cm, preferably from 0.5 mm to 5 cm. The adequate hydraulic diameter may preferably be a function of the flow rate that will go through the injection channel 7: for example, for a flow rate from 1 to 10 mL/min, the diameter scale is that of the millimeter, while a hydraulic diameter of 1 cm may be suitable for a flow rate from 100 to 1000 mL/min.

For a non-circular injection channel 7, the term *"hydraulic diameter"* is defined as being equal to the ratio of 4xA/P where A is the cross section of the injection channel 7 and P is the perimeter of the injection channel 7.

According to some preferred embodiments, the injection channel 7 comprises only one inlet 3.

According to other embodiments, the injection channel 7 may comprise more than one inlet 3, for example two or three inlets 3. This embodiment is particularly suitable in case many devices are in parallel or in order to avoid having bubbles in the injection channel 7.

The inlet 3 makes it possible to introduce a fluid (such as a composition comprising a precipitation agent) into the injection channel 7.

As mentioned above, the injection device 1 comprises a plurality of outlets Si located on the external surface of the injection channel 7. The injection device 1 may comprise from 2 to 40 outlets, and preferably from 2 to 20 outlets. For example, the injection device 1 may comprise from 2 to 5 outlets Si; or from 5 to 10 outlets Si; or from 10 to 15 outlets Si; or from 15 to 20 outlets Si; or from 20 to 25 outlets Si; or from 25 to 30 outlets Si; or from 30 to 35 outlets Si; or from 35 to 40 outlets Si.

If the injection channel 7 has a rectangular or square (or otherwise polygonal) cross-section, the external surface on which the outlets Si are arranged may be a planar surface. Advantageously, all outlets Si are arranged on one and the same planar surface. In other embodiments, the outlets Si may be arranged on two or more different planar surfaces.

According to some embodiments and as represented in **figure 1**, each outlet Si (S1 to S20 in case of **figure 1**) is simply formed by a hole on a wall of the injection channel 7.

According to other embodiments and as represented in **figure 2****,** each outlet Si (S1 to S20 in case of **figure 2**) is formed as a side conduit branching from the external surface of the channel 7.

Each outlet Si preferably has a circular cross-section, although other shapes such as a square or rectangular cross-section are also possible. The internal diameter of an outlet Si may be defined as the maximum cross-sectional dimension of the outlet (perpendicular to the direction of flow through the outlet).

In some embodiments, different outlets Si may have different internal diameters. In other embodiments, all outlets Si have the same internal diameter.

Advantageously, these respective diameters as well as the length of the injection channel 7 (along the main direction of flow) are selected such that, upon use, the pressure drop generated when the fluid flows through the injection channel 7 is lower than the pressure drop generated when the fluid exits the injection channel 7 via the plurality of outlets Si - preferably lower by a factor of at least 2, more preferably by a factor of at least 3, and even more preferably by a factor of at least 5. This makes it possible to distribute fluid evenly through the various outlets Si.

Preferably, the flow rate through each outlet Si is substantially equal to the total flow rate through the injection channel 7 divided by the total number of outlets Si.

According to some embodiments (notably represented in **figures 1** and **2**), the outlets Si may be arranged along a straight line on the external surface of the injection channel 7. Furthermore, the outlets Si may be regularly spaced along the injection channel 7 (the distance between adjacent outlets Si being constant). This makes it possible to distribute the fluid from the outlets Si in a regular manner.

In other embodiments, the injection device 1 may comprise two or more injection zones 8. Each injection zone 8 comprises two or more, outlets Si. This is notably illustrated in **figures 3** and **4****.**

Outlets in one injection zone 8 are located significantly closer to each other than to outlets in other injection zones 8. This can be mathematically defined for example as follows. Let dsi-sj be the distance between the geometric centers of any two outlets Si and Sj. Let d^{A}_{in max} be the maximum value of d_{Si-Sj} for all possible pairs of outlets Si-Sj wherein both Si and Sj belong to a same injection zone A. Let d^{A}_{out min} be the minimum value of dsi-sj for all possible pairs of outlets Si-Sj wherein Si belongs to the injection zone A and Sj does not belong to the injection zone A. Preferably, d^{A}_{in max} is less than 5 times d^{A}_{out min}. More preferably, d^{A}_{in max} is less than 10 times d^{A}_{out min}. Even more preferably, d^{A}_{in max} is less than 20 times d^{A}_{out min}. Preferably, this condition is satisfied for all injection zones 8.

In some embodiments, each outlet Si is part of an injection zone 8 (as illustrated in **figures 3** and **4**)**.** In other embodiments, some outlets Si may be part an injection zone 8, and one or more other outlets Si may be isolated, i.e. not belong to any injection zone 8.

An uneven distribution of outlets Si on the injection channel 7, and in particular the presence of injection zones 8 as defined above, makes it possible to distribute the fluid in an irregular manner into the mixing device 2. For example, if each outlet Si distributes an equal unitary fraction of the total flow rate (total flow rate divided by the total number of outlets Si), each injection zone 8 distributes a certain fraction of the flow rate depending on the number of outlets Si it contains.

For example, by making reference to **figure 3** or **figure 4****,** the injection channel 7 comprises five injection zones 8: a first injection zone 8 comprising outlets S1 to S6 (which may distribute 30% of the total flow rate of fluid), a second injection zone 8 comprising outlets S7 and S8 (which may distribute 10% of the total flow rate of fluid), a third injection zone 8 comprising outlets S9 to S11 (which may distribute 15% of the total flow rate of fluid), a fourth injection zone 8 comprising outlets S12 to S15 (which may distribute 20% of the total flow rate of fluid) and a fifth injection zone 8 comprising outlets S16 to S20 (which may distribute 25% of the total flow rate of fluid).

The injection channel 7 can comprise from 1 to 40 injection zones 8, and preferably from 5 to 20 injection zones 8.

Each injection zone 8 may comprise from 2 to 40 outlets Si. For example, each injection zone 8 may comprise from 2 to 3; or from 3 to 5; or from 5 to 10; or from 10 to 15; or from 15 to 20; or from 20 to 25; or from 25 to 30; or from 30 to 35; or from 35 to 40 outlets Si.

According to some embodiments (notably when the precipitation agent is polyethylene glycol as will be described below), the injection channel 7 may comprise three injection zones 8 and two isolated outlets. For example, from closer to further from the inlet 3, the outlets Si may be distributed in 1) a zone comprising 12 outlets Si, 2) an isolated outlet Si, 3) another isolated outlet Si, 4) a zone comprising 3 outlets Si and 5) a zone comprising 14 outlets Si. Preferably each zone or isolated outlet represents approximately 20 % of the precipitating yield.

According to other embodiments (notably when precipitation agents different from polyethylene glycol are used), the injection channel 7 may comprise from 3 to 6 outlets Si, the outlets Si being arranged along a straight line regularly spaced along the injection channel 7.

The injection device 1 may comprise at least one additional outlet (other than the outlets Si, and not connected to the mixing device 2). This additional outlet may be used for example to purge or wash the injection device 1, or to remove any gas bubble present in the device. The additional outlet(s) may advantageously be closed when the injection device 1 is operated as described below, so that fluid can flow through the injection device 1 and out from the outlets Si.

The injection device 1 may also comprise one or more pumps for the injection of fluid through the inlet 3 and out from the outlets Si. Advantageously, the injection device 1 comprises a single pump for distributing the fluid through the inlet 3 and out from the outlets Si. This makes it possible to simplify the configuration of the apparatus of the present invention.

In some embodiments, the injection device 1 can include a pressure control device like a hydraulic back regulator, a check valve, or any similar device that can be used to set up a pressure drop on the outlets Si. This embodiment can be particularly suitable for large scale devices.

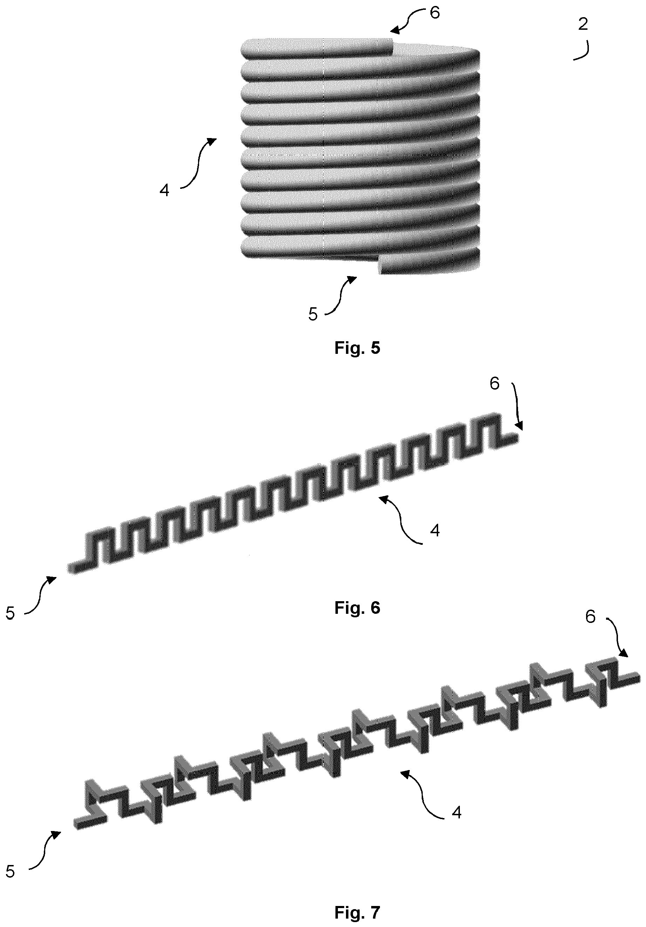

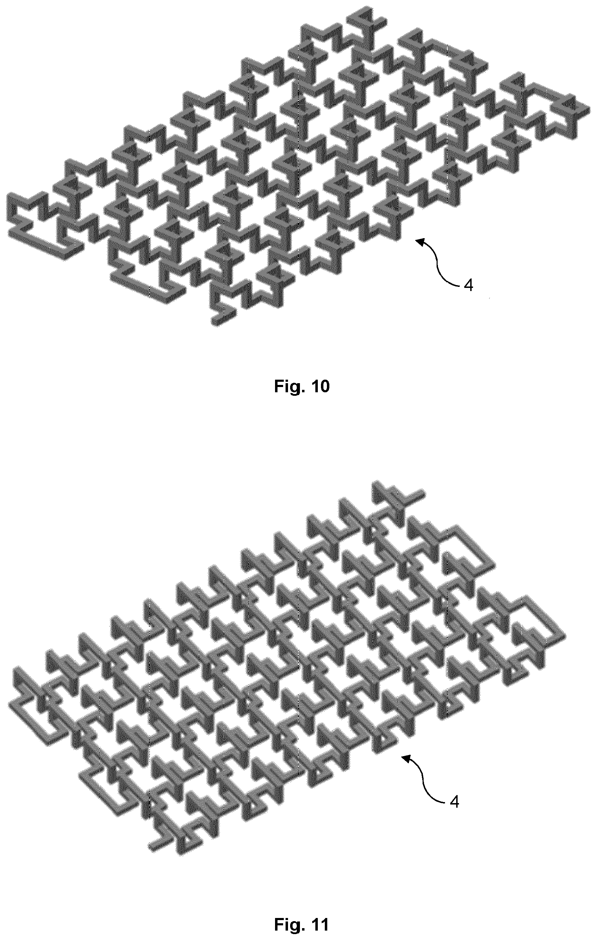

The mixing device 2 can be described by making reference to **figures 5** to **11****.** The mixing device 2 of the present invention comprises a mixing channel 4 being provided with at least one main inlet 5 on a first extremity of the mixing channel 4 and at least one main outlet 6 on a second extremity of the mixing channel 4. By "*channel*" is meant a tubular passage formed in the mixing device 2.

According to some embodiments, the mixing channel 4 is provided with a single main inlet 5 and a single main outlet 6. The main inlet 5 makes it possible to introduce a fluid which is a composition comprising at least one biomolecule of interest and at least one impurity into the mixing channel 4. The main outlet 6 makes it possible to recover the composition obtained after the precipitation by admixing the precipitation agent.

The mixing channel 4 can have for instance a rectangular or square or circular cross-section (perpendicular to the main direction of flow).

The mixing channel 4 may have an internal hydraulic diameter from 0.5 to 50 mm, preferably from 0.8 to 10 mm, and more preferably from 0.9 to 2 mm For example this diameter may be from 0.5 to 1 mm; or from 1 to 2 mm; or from 2 to 4 mm; or from 4 to 6 mm, or from 6 to 8 mm, or from 8 to 12 mm, or from 12 to 15 mm; or from 15 to 20 mm, or from 20 to 25 mm; or from 25 to 30 mm, or from 30 to 35 mm; or from 35 to 40 mm; or from 40 to 45 mm; or from 45 to 50 mm. By way of example, an internal hydraulic diameter of 0.9 to 1 mm may be preferred.

For a non-circular mixing channel 4, the *"hydraulic diameter"* is defined as being the ratio of 4xA/P where A is the cross section of the mixing channel 4 and P is the perimeter of the mixing channel 4. According to some embodiments, and as will be detailed below, the mixing channel 4 can be for example the interior or a tube or a conduit. The tube or conduit can be made for example from steel or polymers such as silicone based plastics, thermoplastic elastomers (TPE), polypropylene, polyvinylidene fluoride (PVDF), polyethylene and polymethyl methacrylate. Alternatively, the mixing channel 4 can be formed for example from a plurality of plates which are assembled together. The plates can be made for example from steel or polymers such as silicone based plastics, TPE, polypropylene, PVDF, polyethylene, polymethyl methacrylate.

According to some embodiments, the mixing device 2 is configured as a conduit coiled around a central axis.

In such embodiments, it is preferable that the mixing channel 4 has a circular cross-section. Such embodiments are preferable when large scale purifications are needed.

The coil may be e.g. configured as a circular helix, as a conical helix or as a flat spiral.

A specific example of such embodiment is illustrated in **figure 5****.** In this case, the coil is configured as a circular helix. As illustrated in **figure 5****,** the main inlet 5 is located at a lower end of the mixing device 2 while the outlet 6 is located at an upper end of the mixing device 2. However, it is also possible to consider that the main inlet 5 is located at the upper end of the mixing device 2 while the main outlet 6 is located at the lower end of the mixing device 2.

Preferably, the volume of the coiled conduit, from the main inlet 5 to the main outlet 6, may be adapted depending on the scale of the apparatus and thus on the flow rate in the coiled conduit.

Concerning the flow rate in the coiled conduit, at laboratory scale, the flow rate may range from 1 to 10 mL/min while for production scale, the flow rate may range from 100 to 500 L/hour. Thus the volume of the coiled conduit may be determined in order to ensure a residence time from 0.5 to 60 minutes, for example a residence time from 0.5 to 3 minutes, or from 3 to 6 minutes, or from 6 to 9 minutes, or from 9 to 12 minutes, or from 12 to 15 minutes, or from 15 to 25 minutes, or from 25 to 35 minutes, or from 35 to 45 minutes, or from 45 to 60 minutes.

According to other embodiments, the mixing channel 4 comprises a plurality of successive channel units. The channel units may be identical, in which case a same pattern is repeated along the channel a plurality of times. Each channel unit may in particular comprise a plurality of intersecting linear segments. Such embodiments are illustrated in **figures 6** to **11****.** They may be preferable when small scale purifications are needed.

Adjacent linear segments of the mixing channel 4 may form an angle of from 20 to 150°. Preferably this angle can be from 30 to 100°, and more preferably 90°. The pattern can be repeated from 2 to 10000 times, preferably from 50 to 5000 and more preferably from 100 to 1500 times. The resulting tortuosity of the mixing channel 4 makes it possible to mix the components present in the mixing channel 4 in an efficient manner. More particularly, notably when the fluids having different viscosities are mixed, said tortuosity makes it possible to reduce or prevent the accumulation of the more viscous fluid which would otherwise tend to accumulate in the lower parts of the mixing channel 4.

The linear adjacent segments may preferably all have the same length (along the main direction of flow). Alternatively, some linear adjacent segments may have different lengths.

According to some embodiments, the mixing channel 4 may comprise a single repeating pattern.

According to other embodiments, the mixing channel 4 may comprise more than one repeating patterns.

According to some embodiments, such as the one illustrated in **figure 6****,** the configuration of the mixing channel 4 is two-dimensional, i.e. all segments belong to the same plane.

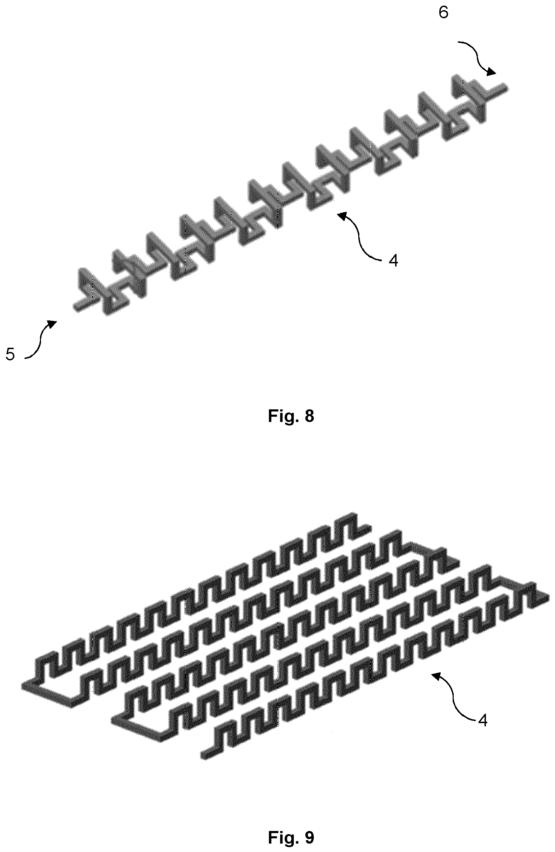

According to other embodiments, such as the embodiments illustrated in **figures 7** and **8****,** the configuration of the mixing channel 4 is three-dimensional, i.e. not all segments belong the same plane.

The mixing channel 4 may also comprise several portions, each portion being patterned as described above. The various portions may be disposed parallel to each other, and connected together. This may increase the compacity of the structure.

For example, **figures 9** to **11** illustrate channels having the same patterns as those illustrated in **figures 6** to **8** respectively, but this time distributed in several successive portions positioned in parallel and connected together.

Other possible structures, not illustrated in the figures, include the superposition of two or more configurations illustrated in **figures 9** to **11****,** such structures still forming a single mixing channel 4.

In addition, making reference to all possible mixing devices 2 according to the invention, other possible structures, not illustrated in the figures, include positioning at least two mixing channels 4 (for example at least two mixing channels 4 according to **figure 5****,** or at least two mixing channels 4 according to **figure 6** to **8**) in series. In this case, each mixing channel 4 can be connected to a different injection device 1 making it possible to inject a different precipitation agent into each mixing channel 4.

The mixing device 2 can also comprise at least one pump. The pump makes it possible to move the mixture within the mixing channel 4.

In addition, when the mixing device 2 is configured as a coiled conduit, the apparatus may comprise a static mixer combined with the mixing device 2.

As mentioned above, the injection device 1 is connected to the mixing device 2. More particularly, the plurality of outlets Si of the injection device 1 are connected to respective secondary inlets of the mixing channel 4. This makes it possible to directly inject the precipitation agent into the mixing channel 4, and preferably by using a single pump. In addition, the fact that the injection device 1 comprises a plurality of outlets Si makes it possible to continuously inject the precipitation agent in different locations into the mixing channel 4.

The outlets Si of the injection device 1 can be connected directly to the secondary inlets of the mixing channel 4. By *"directly"* is meant without the intermediate of tubes or conduits. This is the case, for example, for the injection device illustrated in **figure 2****,** wherein each outlet Si forms a side conduit which may lead to inside the mixing channel 4. Alternatively, the secondary inlets of the mixing channel can be in the form of side conduits. Alternatively, the outlets Si of the injection device 1 can be indirectly connected (owing to e.g. intermediate of tubes or conduits) to the mixing channel 4. This can be the case, for example, for the injection device illustrated in **figure 1****,** wherein each outlet Si is a simple hole. Still alternatively, both the outlets Si of the injection channel 7 and the secondary inlets of the mixing channel 4 may be simple holes put in contact with one another.

In case the mixing device 2 comprises a coiled conduit (as illustrated for example in **figure 5**) each of the outlets Si of the injection device 1 can be connected to a different coil of the channel 4 of the mixing device 2. For example, the injection device 1 of **figure 1** or **2** can be used to inject a precipitation agent into the mixing device 2 of **figure 5****.** In this case, the injection of the precipitation agent into different coils of the mixing device 2 of **figure 5** through the outlets Si of the injection device 1 of **figure 1** or **2** is made possible due to linear configuration of the outlets Si on the surface of the injection device 1. Alternatively, more than one outlet Si can be connected to each coil of the channel 4 of the mixing device 2. For example, the injection device 1 of **figure 3** can be used to inject a precipitation agent into the mixing device 2 of **figure 5****.** In this case, a different amount of precipitation agent can be injected into various coils, this amount depending on the number of outlets Si connected to each coil.

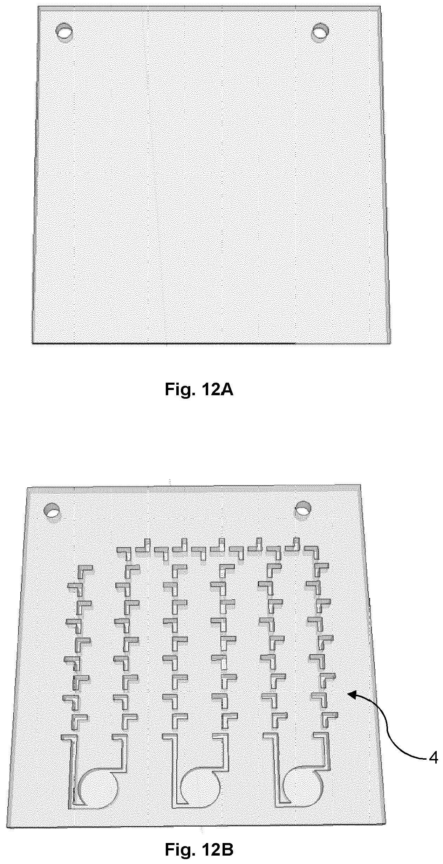

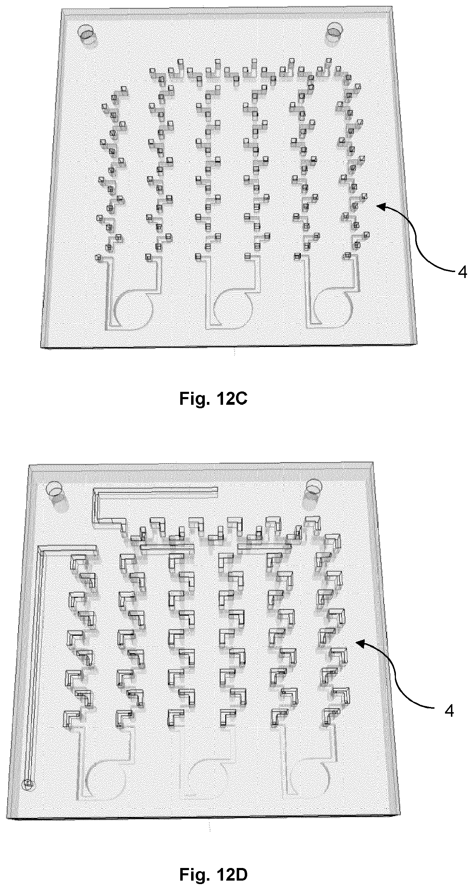

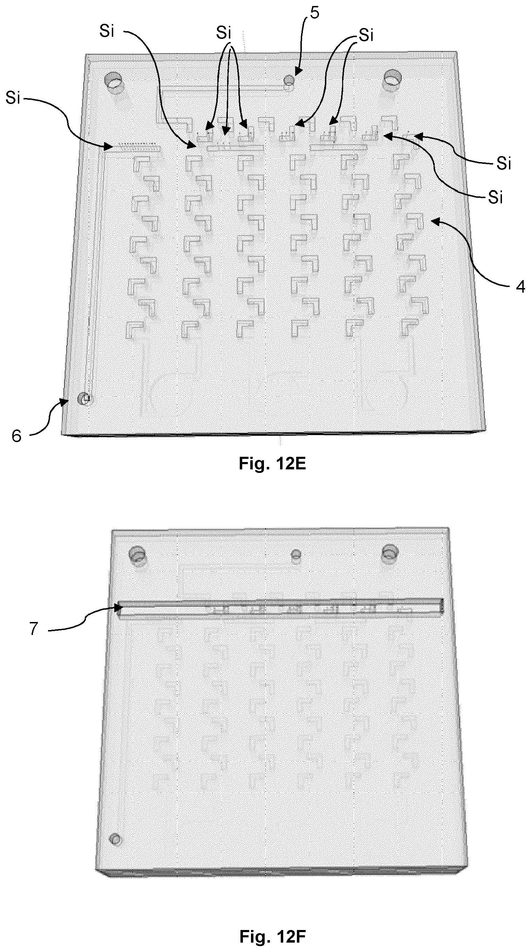

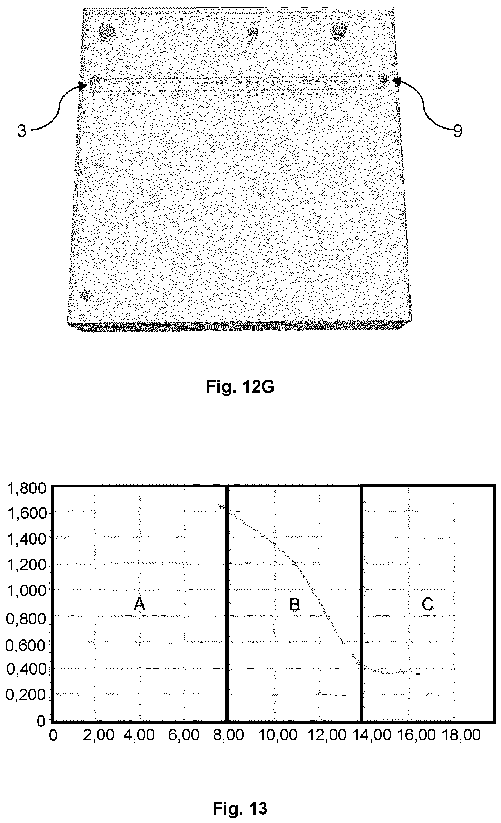

According to some embodiments, notably in case the mixing channel 4 is formed of a plurality of intersecting linear segments, the apparatus can be made by a plurality of plates comprising cutouts, which are tightened and assembled together. Such a configuration is illustrated in **figures 12A** to **12G** wherein the plates of **figures 12A** to **12G** are assembled in the order from the plate of **figure 12A** **to** the plate of **figure 12G****.** More specifically, the plates of **figures 12B** to **12D** comprise cutouts which, when assembled, form the mixing channel 4; the plate of **figure 12E** comprises cutouts for the side conduits corresponding to the outlets Si of the injection device 1; the plate of **figure 12F** comprises cutouts for the injection channel 7; and the plate of **figure 12G** comprises cutouts for the inlet 3 of the injection channel 7 and for an additional outlet 9 (other than the outlets Si) which may be generally closed but may also be used to evacuate air if necessary (it goes without saying that the position of the inlet 3 and the secondary outlet 9 can be inversed on the plate.). Thus, the apparatus includes a mixing channel 4 which is provided with a main inlet 5 on the upper part of the plate of **figure 12E** for the introduction of a fluid (for example the mixture comprising at least one biomolecule of interest and at least one impurity) and a main outlet 6 on the lower part of the plate of **figure 12E** for the withdrawal of the final solution (after precipitation). It goes without saying that the position of the main inlet 5 and the main outlet 6 can be inverted on the plate.

The mixing channel 4 illustrated in **figures 12B** to **12D** comprises a repeating pattern (as detailed above). In this specific configuration, the mixing channel 4 comprises multiple portions connected in parallel. In addition, **figure 12E** illustrates a plurality of outlets Si (belonging to the injection channel 7 illustrated in **figure 12F**) connected to different positions of the mixing channel 4.

In some embodiments, the mixing channel 4 may comprise yet additional inlet(s), preferably a single additional inlet. The additional inlet(s) may be connected to a source other than the injection device 1 and the source of mixture to be purified. Such additional inlet may make it possible to introduce an additional component into the mixing channel 4 (such as a solubilization agent or a buffer solution). The additional inlet may preferably be located downstream from the main inlet 5, and more preferably closer to the main outlet 6 of the mixing channel 4 than to the main inlet 5 (along the direction of flow). In some embodiments, the additional inlet is located between two secondary inlets.

### Method

As mentioned above, the invention relates to a method for the purification of at least one biomolecule from a composition comprising at least one biomolecule and at least one impurity. Preferably, this method is carried out with the apparatus described above.

The method according to the invention comprises a first step of injecting a composition comprising at least one biomolecule and at least one impurity into the mixing channel 4 via the main inlet 5 of the mixing device 2. This composition is preferably an aqueous composition.

The biomolecule may be a protein, a nucleic acid or an antibody.

According to preferred embodiments, the biomolecule is an antibody. The antibody may for example be a monoclonal or polyclonal antibody.

The impurity may be chosen from double stranded DNA (dsDNA), host cell proteins (HCP), high molecular weight impurities (HMWI), low molecular weight impurities (LMWI), and antibody fragments or aggregates.

Thus, the composition to be injected into the mixing channel 4 of the mixing device 2 may derive from a cell culture, or from a fermentation process (for example from a fermentation or a clarified broth) or from a sample of natural fluid such as a blood sample for example.

The ratio of the flow rate of the precipitation agent to the flow rate of the composition injected into the mixing channel 4 may be from 1 to 60%. This ratio may notably be from 1 to 3 %, or from 3 to 6 %, or from 6 to 9 %, or from 9 to 12 %, or from 12 to 15 %, or from 15 to 20 %, or from 20 to 30 %, or from 30 to 40 %, or from 40 to 50 %, or from 50 to 60%.

The method further comprises a step of injecting a composition comprising at least one precipitation agent into the mixing channel 4. This composition is preferably an aqueous composition.

Preferably, both injections are performed continuously. Alternatively, notably in case of cleaning or start-up procedures, it may be interesting to stop the injection of the precipitation agent or the injection of the composition to be purified.

Therefore, while the composition comprising at least one biomolecule and at least one impurity is injected and therefore flows in the mixing channel 4, the composition comprising at least one precipitation agent is injected in a continuous manner into the mixing channel 4 at different positions and therefore continuously comes into contact with the composition comprising at least one biomolecule and at least one impurity at these different locations.

By *"precipitation agent"* is meant a compound capable of precipitating (forming a solid phase in the liquid mixture) one of the biomolecule or the impurity(ies) comprises in the mixture described above. The precipitation agent may be chosen from a charged polymer, a cationic detergent, a short-chain fatty acid, an inorganic salt and their combinations. For example, the precipitation agent may be chosen from a fatty acid, in particular caprylic acid (for example at a concentration of 1 to 4 % w/v), polyethylene glycol (PEG) of different molecular weights (*e.g*. from 100 to 50000 or from 300 to 20000, for example approximately 300, or approximately 600, or approximately 1500, or approximately 4000, or approximately 6000, or approximately 20000), calcium chloride (for example having a concentration of 100 to 500 mM)zinc chloride, alcohols such as ethanol and isopropanol, as well as their combinations.

According to some embodiments, the above composition comprises a single precipitation agent.

According to other embodiments, the above composition comprises a mixture of different precipitation agents. For example, a salt (such as calcium chloride) may be combined with a fatty acid (such as caprylic acid).

According to other embodiments, the addition of the precipitation agent is carried out after a pH adjustment with a buffer, such as a phosphate buffer (for example 2 mM and higher).

As a result, mixing of the precipitation agent with the composition comprising at least one biomolecule and at least one impurity results in the precipitation of one compound in the mixing device 2. According to some embodiments, this compound may be the biomolecule comprised in the composition comprising at least one biomolecule and at least one impurity. In this case, the biomolecule being in a solid state can be isolated and separated from the impurity, downstream from the mixing device 2. According to other embodiments, this compound may be the impurity comprised composition comprising at least one biomolecule and at least one impurity. In this case, the impurity being in a solid state, it can be removed and the liquid phase comprising the biomolecule may be recovered and isolated, downstream from the mixing device 2. In both cases, separation may be performed for example by filtration and/or centrifugation.

The composition comprising the precipitation agent may then be injected into the mixing channel 4 via the outlets Si present on the surface of the injection channel 7. This makes it possible to continuously distribute the composition comprising the precipitation agent at different positions (locations) into the mixing channel 4. The number and position of injection zones 8 on the injection channel 7 and also the number of outlets Si in each injection zone 8 make it possible to control the distribution and thus the precipitation of either the biomolecule or the impurity.

For example, by making reference to **figure 13****,** which illustrates the solubility curve of an antibody during the addition of PEG 6000 as a precipitation agent, three areas (A, B, C) can be distinguished. The first area (A) corresponds to a zone wherein, despite the injection of the precipitation agent, precipitation of the antibody has not yet started. The second area (B) corresponds to a zone wherein rapid precipitation follows the addition of the precipitation agent. Finally, the third area (C) corresponds to a zone wherein precipitation of the antibody has almost ended, and injection of the precipitation agent will only precipitate a minor amount of antibody. It is preferable that the injection of the precipitation agent should be adapted to the solubility curve illustrated in **figure 13****.** For example, when carrying out the purification in an apparatus comprising the injection device 1 illustrated in **figure 3** and the mixing device 2 illustrated in **figure 5** and by making reference to **figure 13****,** a first amount of precipitation agent may be injected through outlets S1 to S6 (this corresponds to area A of **figure 13**), then precipitation may continue slowly by injecting the precipitation agent through outlets S7, S8, then outlets S9 to S11, and then outlets S12 to S15 (this corresponds to area B of **figure 13****,** wherein the precipitation agent should be added at a lower rate in order to avoid oversaturation) and finally precipitation is terminated by injecting the precipitation agent through channels S16 to S20 (this corresponds to area C of **figure 13**).

According to some embodiments, the method according the invention may comprise a step of injecting a composition comprising a first precipitation agent and then a step of injecting a composition comprising a second precipitation agent. Both injections are preferably continuous. This can be carried out when two injection devices 1 are connected to the mixing device 2. Thus, a first precipitation agent can be injected through the outlets Si of the first injection device 1 while a second precipitation agent can be injected through the outlets Si of the second injection device 1. It goes without saying that more than two precipitation agents may be injected in the same way. Alternatively, this can be carried out when two mixing devices 2 are connected in series, each one being connected with a different injection device 1 (and each injection device 1 injecting a different precipitation agent). This makes it possible to firstly precipitate a first impurity by using a first precipitation agent, and then precipitate a second impurity by using a second precipitation agent. Alternatively, it is possible to firstly precipitate an impurity with a first precipitation agent and then precipitate the biomolecule of interest with a second precipitation agent.

According to some embodiments, the method according to the invention may further comprise a step of injecting a solubilization agent into the mixing channel 4. This injection is preferably continuous. The purpose of injecting a solubilization agent may be to solubilize small particles prior to re-precipitating a further amount of impurity or biomolecule based on the growth phenomenon rather than the nucleation phenomenon. The solubilization agent may be chosen from state-of-the-art buffers, such as water, ultrapure water, phosphate buffer (PBS) or glycine. The injection of such agent may be carried out relatively close to the end of the precipitation; for example it may be performed just upstream the last injection zone, or upstream the two last injection zones.

According to some embodiments, the method according to the invention may further comprise a step of injecting a buffer solution into the channel 4. This injection is preferably continuous. The purpose of injecting such buffer solution may be to modify the pH during the precipitation in order to avoid damage of the biomolecule of interest.

## Claims

1. An apparatus for the purification of biomolecules, comprising:
- an injection device (1) comprising an injection channel (7), the injection channel (7) being provided with at least one inlet (3) for a precipitation agent composition and a plurality of outlets (Si) on the external surface of the injection channel (7); and
- a mixing device (2) comprising a mixing channel (4) provided with at least one main inlet (5) for a composition to be purified and at least one main outlet (6), as well as a plurality of secondary inlets;
wherein the injection device (1) and the mixing device (2) are coupled such that each of the plurality of outlets (Si) of the injection device (1) is connected to a respective secondary inlet of the mixing channel (4).

2. The apparatus according to claim 1, wherein the injection channel (7) comprises from 2 to 40 outlets (Si), and preferably from 2 to 20 outlets (Si).

3. The apparatus according to any one of claims 1 or 2, wherein the outlets (Si) are distributed substantially linearly on the external surface of the injection channel (7) and are preferably evenly spaced.

4. The apparatus according to any one of claims 1 or 2, wherein the outlets (Si) are grouped as distinct injection zones (8) distributed along the injection channel (7).

5. The apparatus according to any one of claims 1 to 4, wherein the mixing device (2) is configured as a coiled conduit.

6. The apparatus according to any one of claims 1 to 4, wherein the mixing channel (4) is formed of a plurality of intersecting linear segments.

7. The apparatus according to any one of claims 1 to 6, comprising only one pump connected to the injection device (1).

8. A set comprising the apparatus according to any one of claims 1 to 7, a composition comprising at least one biomolecule and at least one impurity, and a composition comprising at least one precipitation agent.

9. The set according to claim 8, wherein the biomolecule is chosen from a protein and a nucleic acid and is preferably an antibody; and/or wherein the impurity is chosen from double stranded DNA, host cell proteins, high molecular weight impurities, low molecular weight impurities, and antibody fragments or aggregates; and/or wherein the precipitation agent is chosen from caprylic acid, polyethylene glycol, calcium chloride, zinc chloride, ethanol, isopropanol and combinations thereof.

10. A method for the purification of biomolecules using the apparatus of any one of claims 1 to 7 comprising:
- the injection of a composition comprising at least one biomolecule and at least one impurity into the mixing channel (4) via the main inlet (5);
- the injection of a composition comprising at least one precipitation agent from the injection device (1) into the mixing channel (4) via the plurality of outlets (Si) of the injection device (1); and
- the precipitation of one of the at least one biomolecule or the at least one impurity in the mixing device (2).

11. The method according to claim 10 wherein both the injection of the composition comprising the biomolecule and the impurity and the injection of the composition comprising the precipitation agent are continuous.

12. The method according to any one of claims 10 or 11, wherein the biomolecule is chosen from a protein and a nucleic acid and is preferably an antibody; and/or wherein the impurity is chosen from double stranded DNA, host cell proteins, high molecular weight impurities, low molecular weight isoforms and antibody fragments or aggregates.

13. The method according to any one of claims 10 to 12, wherein the precipitation agent is chosen from caprylic acid, polyethylene glycol, calcium chloride, zinc chloride, ethanol, isopropanol, and combinations thereof.

14. The method according to any one of claims 10 to 13, further comprising a step of injecting into the mixing channel (4) a solubilization agent or a buffer solution via at least one additional inlet of the mixing channel (4).

15. The method according to any one of claims 10 to 14, further comprising a step of injecting a composition comprising at least one additional precipitation agent into the mixing channel (4) via at least two outlets (Si) of an additional injection device (1).
